(19) **Europäisches Patentamt**
**European Patent Office**
**Office européen des brevets**

(11) **EP 3 258 223 B1**

(12) **EUROPÄISCHE PATENTSCHRIFT**

(45) Veröffentlichungstag und Bekanntmachung des Hinweises auf die Patenterteilung:
**22.10.2025 Patentblatt 2025/43**

(51) Internationale Patentklassifikation (IPC):
*G01F 11/00* (2006.01)  *A61M 5/168* (2006.01)
*A61M 5/142* (2006.01)  *G01F 11/04* (2006.01)

(21) Anmeldenummer: **17179898.6**

(22) Anmeldetag: **26.02.2008**

(52) Gemeinsame Patentklassifikation (CPC):
**A61M 5/14216; A61M 5/152; A61M 5/16809; G01F 11/04**

(54) **DOSIERVORRICHTUNG FÜR EIN INFUSIONSSYSTEM**

DOSING DEVICE FOR AN INFUSION SYSTEM

DISPOSITIF DE DOSAGE POUR UN SYSTÈME D'INFUSION

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **15.03.2007 EP 07104240**

(43) Veröffentlichungstag der Anmeldung:
**20.12.2017 Patentblatt 2017/51**

(62) Dokumentnummer(n) der früheren Anmeldung(en) nach Art. 76 EPÜ:
**15165236.9 / 3 012 600**
**08716055.2 / 2 118 624**

(73) Patentinhaber:
• **F. Hoffmann-La Roche AG**
  **4070 Basel (CH)**
• **Roche Diabetes Care GmbH**
  **68305 Mannheim (DE)**
  Benannte Vertragsstaaten:
  **DE**

(72) Erfinder:
• **HAUETER, Ulrich**
  **3506 Grosshöchstetten (CH)**
• **KUEHNI, Florian**
  **3400 Burgdorf (CH)**

(74) Vertreter: **SSM Sandmair**
**Patentanwälte Rechtsanwalt**
**Partnerschaft mbB**
**Joseph-Wild-Straße 20**
**81829 München (DE)**

(56) Entgegenhaltungen:

| | |
|---|---|
| EP-A1- 1 754 505 | EP-A2- 0 980 690 |
| EP-A2- 1 486 218 | WO-A1-2007/000064 |
| WO-A1-93/20864 | WO-A1-93/20864 |
| DE-A1- 3 832 028 | DE-A1- 3 832 028 |
| US-A- 3 631 654 | US-A- 3 631 654 |
| US-A- 5 807 321 | US-A- 5 807 321 |
| US-A- 5 961 303 | US-A- 5 961 303 |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

# EP 3 258 223 B1

**Beschreibung**

Die vorliegende Erfindung bezieht sich auf eine Dosier- und Fördervorrichtung und insbesondere auf eine Vorrichtung zum Dosieren einer zum Beispiel mittels eines Infusions-Sets an einen Patienten abzugebende Substanz.

Aus der CH 688 224 A5 ist eine implantierbare Vorrichtung für die dosierte Abgabe pharmazeutischer Flüssigkeiten im menschlichen oder tierischen Körper bekannt, wobei die implantierbare Einheit eine kurvengesteuerte, ventillose Axialkolbenpumpe mit einem drehantreibbaren und axial verschiebbaren Kolben, ein mit der Saugseite der Pumpe verbundenes Flüssigkeitsreservoir und einen steuerbaren, mit dem Kolben verbundenen Drehantrieb aufweist. Dabei ist an einem unteren Ende eines Zylinders je eine Saugöffnung und ein Drucköffnung angeordnet, wobei die beiden Öffnungen sich diametral gegenüberliegen und gleichachsig verlaufen.

Die US 6,010,485 offenbart einen Arbeitszylinder mit einem ähnlichen Aufbau wie in der CH 688 224 A5.

Die US 6,749,587 B2 offenbart ein modulares Infusionsgerät mit einem Messabschnitt, welcher direkt den Fluidfluss zwischen einem Reservoir und einer Kanüle steuern kann. Wenn das Reservoir bei Umgebungsdruck gehalten wird, kann der Messabschnitt einen Peristaltik-Mechanismus, eine Verdrängerpumpe oder eine andere Pumpvorrichtung aufweisen.

Aus der US 4,643,723 ist eine Vorrichtung zur Verabreichung von Insulin an einen Patienten bekannt, wobei in einer Pumpkammer ein Kolben angeordnet ist, eine Kanüle mit der Pumpkammer verbunden ist und ein Kolbenstab mit dem Kolben verbunden ist. Wenn der Kolben zurückgezogen wird, wird das Reservoir mit der Pumpkammer verbunden, um die Pumpkammer aufzufüllen und wenn der Kolben nach vorne bewegt wird, wird mittels eines Ventils der Durchgang vom Reservoir zur Pumpkammer geschlossen, so dass die Substanz über eine Kanüle verabreicht werden kann.

Aus der WO 93/04714 und der korrespondierenden EP 0 600 948 B1 ist ein Fluidmesselement für ein implantierbares Verabreichungssystem bekannt, welches zwischen einer unter Druck gesetzten Fluidquelle und einer Auslassöffnung gekoppelt ist, um diskrete Flussimpulse mit einer vorgegebenen Rate zur Verfügung zu stellen.

Die US 2004/0069044 A1 offenbart eine Vorrichtung zum Messen eines Volumens eines Arzneimittels. Die Vorrichtung weist eine erste Kammer, welche das flüssige Arzneimittel enthält, eine Messkammer, die in Fluidverbindung mit der ersten Kammer ist und eine Messanordnung auf.

Die US 5,207,666 offenbart ein Fluidmessgerät für implantierbare Arzneiverabreichungssysteme, welches zwischen einer unter Druck gesetzten Fluidquelle und einer Auslassöffnung angeordnet werden kann, um diskrete Flussimpulse mit einer vorgegebenen Rate zur Verfügung zu stellen.

Aus der US 2005/0159708 A1 ist eine Infusionspumpe zur dosierten Verabreichung einer Flüssigkeit bekannt, wobei ein Kolben permanent mittels einer Feder mit Druck beaufschlagt wird und auf einen Medikamentenbehälter einwirkt, der durch eine Abgabeöffnung die Substanz in Richtung auf ein Steuerventil zum Dosieren abgibt.

Die EP 1 633 417 B1 offenbart ein Abgabegerät mit einer Aufbewahrungskammer und einer Injektionskammer, die über eine Fluidverbindung gekoppelt sind und in welchen jeweils Tauchkolben angeordnet sind.

Aus der US 5,807,321 ist ein System zum elektronischen Überwachen der Verabreichung eines Kontrastmediums bekannt, wobei ein Fluidzylinder beweglich in einer zylinderförmigen Kammer gelagert ist. Durch Drehung des Fluidzylinders, welcher Ventilöffnungspaare aufweist, die mit korrespondierenden Öffnungspaaren des Gehäuses der zylinderförmigen Kammer zusammenwirken, können unterschiedliche Flussrichtungen realisiert werden. Ein innerhalb des Fluidzylinders frei bewegbarer Fluidverdrängungsindikator wird in der Fluidkammer des Fluidzylinders bewegt, wenn ein Fluiddruck an dem ersten Ende oder dem zweiten Ende angelegt wird.

Aus der WO 2007/000064 A1 ist eine Dosiervorrichtung gemäß dem Oberbegriff des Anspruchs 1 bekannt, wobei ein Kolben entgegen der Federkraft einer Feder durch Zuführen einer unter Druck stehenden Flüssigkeit verschoben wird, bis er formschlüssig an einer Stufe des Verdrängungskanals anschlägt, so dass bei dieser Kolbenposition ein genau definiertes maximales Volumen durch den dann entstehenden ersten Verdrängungsraum gebildet wird.

Aus der EP 0 980 690 A2 ist eine Vorrichtung für eine abgemessene Fluidzufuhr bekannt, wobei ein Kolben durch ein unter Druck gesetztes Fluid zwischen zwei Positionen hin- und hergeschoben wird.

Aus der US 5 961 303 A ist eine Pumpe bekannt, die kontrollierte Mengen an fließfähigem Material durch Computersteuerung ausgibt, die sowohl die Verschiebung als auch die Winkelposition des Kolbens mit einem Servomechanismus steuert. Das System umfasst Behältermittel, um einen Vorrat an fließfähigem Produktmaterial, das ausgegeben werden soll, zu halten; eine zylindrische Kolben- und Kolbengehäuseeinrichtung mit einer Vielzahl von Einlaßöffnungseinrichtungen, die das fließfähige Produktmaterial von der Behältereinrichtung aufnehmen können, und mehrere Ausgangsöffnungseinrichtungen, die das Produkt aus dem Behälter ausgeben können; Kolbenstangenmittel, die mit den Kolbenmitteln verbunden sind, um der Kolbeneinrichtung eine lineare und eine Drehbewegung zu verleihen. Eine Dreh- und Linearantriebseinrichtung ist mit der Kolbenstangeneinrichtung verbunden und dazu geeignet, abwechselnd Oberflächenöffnungen mit der Einlaßöffnungseinrichtung auszurichten, während Oberflächenstege den Fluß von den Auslaßöffnungseinrichtungen beschränken und dann den Prozeß zyklisch umkehren. Ein Computer steuert alle Aspekte der Abgabepumpe, einschließlich Rate und Volumen des Wiederauflade- und Entladezyklus, Zeitpunkt, Öffnungsgröße und Geschwindigkeit des Öffnens und Schließens der Steuerventile. In ihrer einfachsten Form benötigt die beschriebene Pumpe nur drei Teile, um mit den gepumpten Bestandteilen in Kontakt zu sein.

Es ist eine Aufgabe der vorliegenden Erfindung, eine Dosiervorrichtung für ein Infusionssystem vorzuschlagen, welche ein einfaches und genaues Dosieren einer abzugebenden Substanz ermöglicht.

Diese Aufgabe wird durch eine Dosiervorrichtung gemäß Anspruch 1 gelöst. Vorteilhafte Ausführungsformen ergeben sich aus den abhängigen Ansprüchen.

Eine Dosiervorrichtung für ein Infusionssystem weist eine Dosiereinheit mit einem veränderbaren Volumen, wie zum Beispiel einen Zylinder mit einem sich in dem Zylinder bewegenden und das Volumen vergrößernden oder verkleinernden Kolben auf. Die Dosiervorrichtung hat einen Zufuhr- und einen Abgabeanschluss, welcher vorzugsweise durch einen einzigen Anschluss, wie zum Beispiel eine einzige Öffnung, z.B. am Zylinder oder an einem Anschlussteil oder an einer Hülse, gebildet wird und ist ansonsten vorzugsweise geschlossen. Durch die Zufuhr- und Abgabeöffnung kann das veränderbare Volumen der Dosiereinheit mit der abzugebenden Substanz zum Beispiel aus einem Vorratsbehälter, wie zum Beispiel einer Ampulle, gefüllt werden, wenn sich das Volumen der Dosiereinheit vergrößert. Wird das veränderbare Volumen der gefüllten Dosiereinheit wieder verkleinert, kann durch die Abgabeöffnung die dosiert abzugebende Substanz wieder ausgegeben werden. Bevorzugt ist die Dosiervorrichtung so ausgebildet, dass der Zufuhr- und Abgabeanschluss, also zum Beispiel die einzige Öffnung der Dosiereinheit, bevorzugt alternierend mit einem Vorratsbehälter, wie zum Beispiel einer Ampulle, und einer Abgabe- oder Verabreichungseinheit, wie zum Beispiel einem Infusions-Set, verbunden werden kann.

Erfindungsgemäß kann somit ein Leck oder ein unbeabsichtigtes und unkontrolliertes Durchtreten einer Substanz aus einem Reservoir zu einer Abgabeeinheit, wie einem Infusions-Set, verhindert werden, da die Dosiereinheit entweder nur mit dem Reservoir oder nur mit der Verabreichungsvorrichtung gekoppelt ist. Das Reservoir ist somit von der Verabreichungseinheit vollständig entkoppelt, also das Reservoir ist vorteilhaft nie direkt mit dem Ausgang verbunden.

Der Zufuhr- und Abgabeanschluss kann auch durch mehrere Öffnungen gebildet werden, welche dann immer so mit einem externen Anschluss verbunden sein sollten, dass die Dosiereinheit durch alle oder einen Teil der Öffnungen entweder nur befüllt oder nur entleert wird, also ein gleichzeitiges Aufnehmen und Abgeben einer Substanz nicht möglich ist. Das oder die gleichzeitig als Einlass oder Auslass wirkenden Verbindungen oder Zugänge oder Löcher der Dosiereinheit ist beziehungsweise sind so koppelbar, dass sie entweder mit einem Reservoir gekoppelt werden können und somit als Einlass für die Dosiereinheit wirken, oder mit einer Verabreichungsvorrichtung, wie zum Beispiel einem Infusionsgerät, gekoppelt werden können, und so als Auslass für die Dosiereinheit wirken. Vorzugsweise sind die Anschlüsse der Dosiereinheit verschlossen, wenn keine Substanz aufgenommen oder abgegeben wird.

Es ist auch möglich, in einer Ruheposition zwischen einer Abgabe und Aufnahme einer Substanz die Öffnungen aktiv z.B. durch ein verschiebbares Dichtelement zu verschließen.

Beispielsweise kann die Dosiereinheit verschiebbar oder drehbar sein, um je nach Verschiebeposition oder Drehstellung mit einem von zwei oder mehr externen Anschlüssen zur Befüllung der Dosiereinheit und zur Abgabe und Weiterleitung der von der Dosiereinheit abgegebenen Substanz verbunden zu werden. Ebenso können auch einer oder mehrere der externen Anschlüsse verschiebbar oder drehbar an der Dosiereinheit vorgesehen sein, so dass die verschiebbaren Anschlüsse zum Beispiel abwechselnd mit der Zufuhr- und Abgabeöffnung der Dosiereinheit verbunden werden. Somit kann eine Ventilwirkung durch eine Art Umschaltventil erhalten werden.

Vorteilhaft ist ein Motor vorgesehen, mit welchem die Dosiereinheit oder der Zufuhr- und Abgabeanschluss der Dosiereinheit bewegt und zum Beispiel gedreht oder verschoben werden kann, wobei der Motor alternativ oder ergänzend auch zur Bewegung, also zum Beispiel zur Verschiebung oder Drehung, der externen Zufuhr- und Abgabeanschlüsse verwendet werden kann, um diese abwechselnd mit der Zufuhr- und Abgabeöffnung der Dosiereinheit zu verbinden. Ebenso kann der oder ein anderer Motor zur Vergrößerung oder Verkleinerung des Volumens zum Beispiel zur Bewegung des Kolbens verwendet werden. Vorzugsweise ist der Kolben des Zylinders so ausgebildet, dass er genau zu einer vorgegebenen Position innerhalb des Zylinders bewegt werden kann, also zum Beispiel exakt zu einer vordefinierten maximalen Auszugsstellung des Kolbens, welche sich vorteilhaft noch innerhalb des Zylinders befindet. Somit kann anders als bei bekannten so genannten "single stroke" Verfahren auch nur ein Teil des veränderbaren (Zylinder-) Volumens zur Dosierung verwendet werden.

Die Verbindungsstellen oder Verbindungsleitungen, welche zu dem Zufuhr- oder Abgabeanschluss der Dosiereinheit führen, weisen vorteilhaft jeweils mindestens ein Ventil auf, wobei auch nur ein einziges Ventil entweder in der Zufuhr- oder in der Abgabeleitung oder in dem Zufuhr- und Abgabeanschluss vorgesehen sein kann. Ein solches Ventil kann in einer oder beiden der Leitungen zum Beispiel als Rückschlagventil sicherstellen, dass eine dosiert abzugebende Substanz nur in eine Richtung gefördert wird, wobei der Rückfluss der Substanz in die Gegenrichtung unterbunden oder verhindert wird.

Das oder die verwendeten Ventile am oder in den Zu- und Ableitungen und/oder an oder in der Dosiereinheit können Rückschlagventile sein, welche einen Fluss einer Substanz oder einer Flüssigkeit nur in eine Richtung ermöglichen oder können auch als Überdruckventile ausgebildet sein, welche einen Durchgang eines Materials oder einer Flüssigkeit erst nach Anliegen eines Mindestdruckes ermöglichen.

Der Vorratsbehälter, welcher die nach Durchlauf durch die Dosiervorrichtung dosiert abzugebende Substanz enthält, kann druckbeaufschlagt oder drucklos sein. Beispielsweise kann der Vorratsbehälter eine Ampulle sein, in welcher ein druckbeaufschlagter Stopfen auf die abzugebende Substanz einwirkt, so dass die abzugebende Substanz nur noch dosiert werden muss und keine weitere Energie für die eigentliche Substanzabgabe aus der Ampulle bzw. das Aufdosieren oder Umfüllen in den Mikrodosierzylinder benötigt wird. Die Druckbeaufschlagung des Stopfens kann beispielsweise mittels einer Feder oder eines unter Überdruck stehenden Gases erfolgen. Ebenso kann der Vorratsbehälter einen elastischen Bereich aufweisen oder vollständig aus einem elastischen Material gebildet sein, wie zum Beispiel ein Beutel, welcher mit der abzugebenden Substanz gefüllt ist. Auf den elastischen Bereich oder Beutel kann zum Beispiel mittels einer Feder eine Kraft oder ein Druck einwirken, um die in dem Vorratsbehälter enthaltene Substanz zu verdrängen und abzugeben, wenn dies durch die dem Vorratsbehälter nachgeschaltete Dosiervorrichtung ermöglicht wird.

Vorzugsweise ist ein Vorratsbehälter oder Reservoir so ausgelegt oder konstruiert, dass bei einem Ausgang oder einer Abgabeöffnung des Reservoirs ein positiver Druck anliegt, was zum Beispiel durch mit Kraft oder Druck beaufschlagte Behälter oder Beutel, oder durch einen aktiven Antriebsmechanismus, welcher auf ein Verdrängungselement des Reservoirs oder das Reservoir selbst einwirkt, erreicht werden kann.

Vorzugsweise weist die Dosiervorrichtung mindestens einen Sensor zur Überprüfung der Funktionsfähigkeit oder zur Feststellung einer Fehlfunktion auf, wie zum Beispiel einen Leck-Sensor, galvanischen oder Leitwertsensor, einen Blasen-Sensor, einen Druck-Sensor oder einen Kraftsensor, welche zum Beispiel feststellen können, ob eine Substanz oder Flüssigkeit aus der Dosiervorrichtung ausgetreten ist, ob zum Beispiel Blasen in der abzugebenden Substanz enthalten sind, oder ob zum Beispiel eine Okklusion vorliegt. Dieser mindestens eine Sensor kann mit einer Warn- oder Alarmanzeige oder einer Steuerung der Dosiervorrichtung verbunden sein, um die Dosiervorrichtung zum Beispiel abzuschalten, wenn eine Fehlfunktion festgestellt wird oder um im Falle einer festgestellten Störung der Dosiervorrichtung ein zum Beispiel optisches oder akustisches Alarmsignal auszugeben.

Vorteilhaft wird ein gas- oder luftdurchlässiges Material für die Dosiervorrichtung und/oder eine Verbindungsleitung der Dosiervorrichtung, wie zum Beispiel eine Zuführleitung oder eine Abgabeleitung, verwendet. Ist in der abzugebenden Substanz Luft oder ein Gas vorhanden, so kann ein zum Beispiel luftdurchlässiger Zuführschlauch, welcher die Dosiervorrichtung mit dem Vorratsbehälter verbindet und somit der Dosierung vorgeschaltet ist, die Möglichkeit schaffen, dass das in der abzugebenden Substanz enthaltene Gas, welches zusammen mit der Substanz durch diese Leitung geführt wird, entweichen kann, um im Idealfall in der Dosiereinheit keine Gaseinschlüsse mehr in der Substanz zu haben.

Vorzugsweise ist an der Dosiervorrichtung eine Dichtung, wie zum Beispiel eine Dichtungsmanschette, vorgesehen, um den Zufuhr- und Abgabeanschluss der Dosiereinheit abzudichten, wenn dieser Anschluss nicht mit einer Zufuhr- oder Abgabeleitung verbunden ist und zum Beispiel mit der Dosiereinheit von der Verbindung mit der Zufuhrleitung zu der Verbindung mit der Abgabeleitung gedreht wird.

Die Dosiervorrichtung ist vorzugsweise so ausgelegt, dass das Maximalvolumen des veränderbaren Volumens der Dosiereinheit $VOL_{Zylinder,\,max}$, also zum Beispiel das Volumen eines Zylinders bei einem fast oder vollständig herausgezogenen Kolben, kleiner ist als das 0,1-fache Volumen des Vorratsbehälters oder eines Reservoirs $VOL_{Reservoir}$, in welchem die gesamte dosiert abzugebende Substanz enthalten ist. Bevorzugt ist das maximale Volumen der Dosiereinheit größer als das 2-fache oder auch z.B. 10-fache des Volumens einer minimal abzugebenden Dosis $VOL_{Dose,\,min}$. Vorzugsweise ist das maximale Volumen der Dosiereinheit gleich dem minimalen Volumen der abzugebenden Dosis multipliziert mit der Wurzel aus dem Quotienten gebildet aus dem Volumen des Reservoirs und dem minimalen Volumen der minimal abzugebenden Dosis und genügt somit der Formel:

$$VOL_{Zylinder,\,max} = VOL_{Dose,\,min} \times \sqrt{(VOL_{Reservoir}/VOL_{Dose,\,min})}$$

Bevorzugt ist die Dosiervorrichtung so ausgelegt, dass:

$$2 \ldots 10 \times VOL_{Dose,min} < VOL_{Zylinder,\,max.} < 1/10 \ldots 1/2\ VOL_{Reservoir}$$

Vorteilhaft ist das Verhältnis des Innendurchmessers einer zylinderförmigen Dosiereinheit zu der Zylinderlänge gleich oder etwa 1:4.

Gemäß einem weiteren Aspekt bezieht sich die Erfindung auf ein Verfahren zur Dosierung einer aus einem Vorratsbehälter oder einem Reservoir abzugebenden Substanz, wobei die Substanz aus dem Vorratsbehälter oder Reservoir über oder durch einen einzigen Anschluss oder eine Öffnung in eine Dosiereinheit mit einem veränderbaren und im Falle der Aufdosierung sich vergrößernden Volumen gebracht wird, der Anschluss oder die Öffnung nach dem Aufdosieren oder Auffüllen des zur Auffüllung sich vergrößernden Volumens mit einer Abgabeleitung verbunden wird und anschließend das die Substanz enthaltende Volumen wieder verkleinert wird, um durch den Anschluss oder diese Öffnung die Substanz dosiert abzugeben.

**EP 3 258 223 B1**

Vorteilhaft wird die Dosiereinheit nach dem Befüllen des veränderbaren Volumens mit der abzugebenden Substanz verschoben oder gedreht, um die Öffnung der Dosiereinheit von der Zufuhrleitung zu entkoppeln und mit der Abgabeleitung zu verbinden.

Erfindungsgemäß kann somit durch eine Dosiervorrichtung unabhängig von der Ausbildung und Größe eines Reservoirs oder eines Vorratsbehälters eine genaue Dosierung der abzugebenden Substanz vorgenommen werden, wobei die Dosiereinheit im Falle der Verwendung einer einzigen Zufuhr- und Abgabeöffnung ein relativ kleines Leck- oder Dichtungsrisiko aufweist.

Die Erfindung wird nachfolgend anhand von Ausführungsbeispielen beschrieben, wobei die beiliegenden Figuren zeigen:

Figuren 1A, B, C    einen prinzipiellen Funktionsablauf beim Dosieren mit der Dosiervorrichtung;

Figur2    eine Ausführungsform der Dosiervorrichtung;

Figur 3    einen Kreisprozess bei einer Ausführungsform der Erfindung; und

Figur 4    einen Kreisprozess bei einer weiteren Ausführungsform der Erfindung.

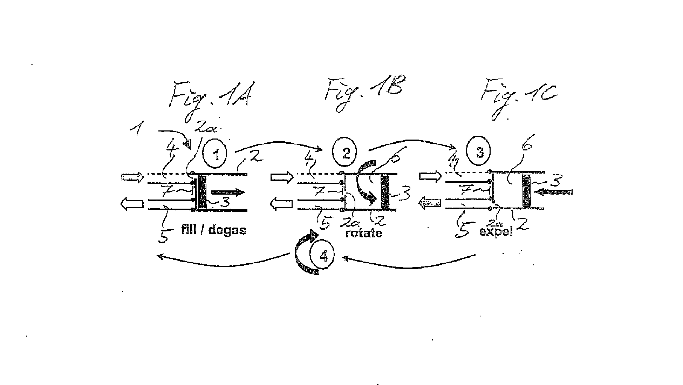

Figur 1A zeigt eine Dosiervorrichtung 1 mit einem Zylinder 2, welcher an einer Stirn- oder Unterseite eine einzige Öffnung 2a aufweist. In der in Figur 1A gezeigten Ausgangslage vor der Befüllung des Zylinders 2 ist die Öffnung 2a mit einer Zuleitung 4 verbunden, welche mit einem nicht gezeigten zum Beispiel unter Druck stehenden Reservoir verbunden ist, das eine dosiert abzugebende Substanz, wie zum Beispiel Insulin, enthält. In dem Zylinder 2 ist ein beweglicher Kolben 3 angeordnet.

Figur 1B zeigt die Dosiervorrichtung 1 gemäß Figur 1A nach dem Aufdosieren der Dosiereinheit 2, 3 durch ein Zurückziehen des Kolbens 3 innerhalb des Zylinders 2, wodurch durch die Zuleitung 4 eine dosiert abzugebende Substanz in das veränderbare Volumen 6 des Zylinders 2 eingebracht wurde. In Figur 1B ist der Zylinder 2 nach dem Abschluss des Befüllvorganges bereits ein Stück um seine Längsachse gedreht worden, wie durch den Pfeil angedeutet, so dass die Öffnung 2a des Zylinders 2 nicht mehr mit der Zuleitung 4 verbunden ist oder in FluidKommunikation mit dieser steht. Die Öffnung 2a ist in dem in Figur 1B gezeigten Zustand in Richtung auf die Ableitung 5 verschoben oder gedreht worden, wobei in dem in Figur 1B gezeigten Zwischenzustand die Öffnung 2a mittels einer an dem Zylinder 2 anliegenden Dichtung 7 verschlossen wird.

Wird der Zylinder 2 so weit gedreht, bis die Öffnung 2a an der Ableitung 5 anliegt, wie in Figur 1C gezeigt, kann durch ein Einschieben des Kolbens 3 in den Zylinder 2 die in dem Volumen 6 enthaltene aufdosierte Substanz durch die Öffnung 2a an die Ableitung 5 abgegeben werden, welche mit einem nicht gezeigten Infusions-Set oder einer Nadel verbunden ist. Nach teilweiser oder vollständiger Abgabe der in dem variablen Volumen 6 des Zylinders 2 enthaltenen Substanz kann der Zylinder 2 zum Beispiel mit vollständig in den Zylinder 2 eingeschobenem Kolben 3 so gedreht werden, dass die Öffnung 2a wieder an der Zuleitung 4 anliegt, wie in Figur 1 durch den Zustand (4) angedeutet, so dass der in Figur 1 gezeigte Zyklus erneut durchlaufen werden kann.

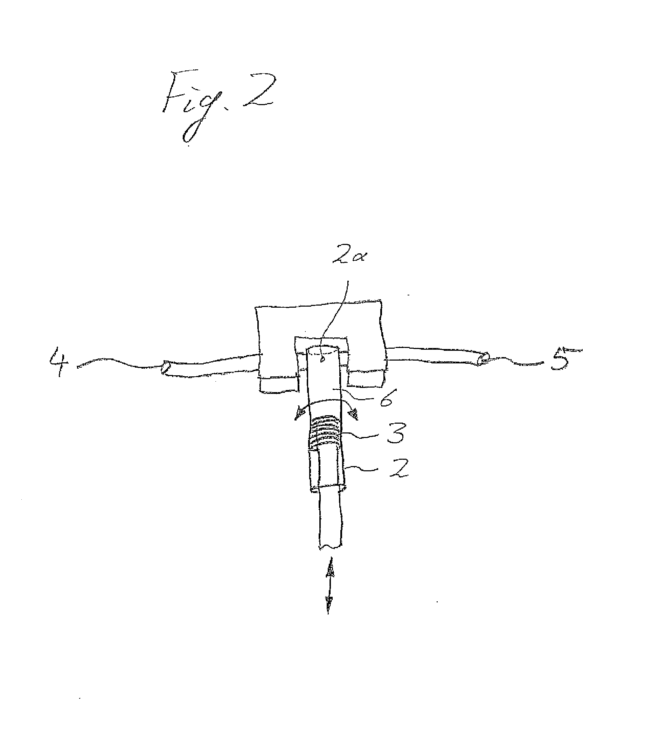

Figur 2 zeigt eine Ausführungsform der erfindungsgemäßen Dosiervorrichtung mit einem aus Kunststoff oder einem z.B. elastischen Material gefertigten drehbaren Zylinder 2, welcher zwischen zwei Leitungen 4, 5 z.B. aus elastischem Material und z.B. auch innerhalb einer Dichthülse mit z.B. zwei Öffnungen angeordnet ist. Durch die in Figur 2 links gezeigte Zuleitung 4 kann das variable Volumen 6 innerhalb des Zylinders 2 durch das Zurückziehen des Kolbens 3 mit einer dosiert abzugebenden Substanz aufgefüllt werden, wenn die an einer seitlichen Oberfläche oder dem Zylindermantel vorgesehene Öffnung 2a in Verbindung mit der Zuleitung 4 ist. Die Öffnung 2a kann ein einfaches Loch in dem Kunststoff-Zylinder 2 sein, welches sich durch die elastische Eigenschaft des Materials des Zylinders 2 verschließt und welches ein Hindurchtreten einer Substanz nur ermöglicht, wenn ein Druck oder Sog auf die Substanz einwirkt, wie zum Beispiel durch das Herausziehen oder Einschieben des Kolbens 3 in den Zylinder 2.

Vorzugsweise ist in der Dosiereinheit mit dem bevorzugt in Zwischenschritten oder stufenweise veränderbaren Volumen der einzige Zufuhr- und Abgabeanschluss asymmetrisch vorgesehen. Ebenso können auch bei zwei oder mehr vorgesehenen z.B. alternierend schließbaren oder öffenbaren Anschlüssen diese Anschlüsse asymmetrisch angebracht sein. Der oder die Anschlüsse können jeweils auch als einfache Öffnung in der Dosiereinheit, wie z.B. einem Zylinder vorgesehen sein. Dabei soll unter dem Begriff "asymmetrischer Anschluss" oder "asymmetrische Öffnung" eine solche Öffnung in der Dosiereinheit oder z.B. einem Zylinder verstanden werden, welche bei einer Bewegung, Verschiebung oder Drehung der Dosiereinheit um eine Symmetrieachse ihre Position verändert. Eine asymmetrische Öffnung kann z.B. an der Stirnseite eines Zylinders vorgesehen sein, sofern sie nicht in der Mitte der Stirnseite liegt, sondern von der Mitte der Stirnseite versetzt ist. Ebenso kann die asymmetrische Öffnung z.B. seitlich an der Dosiereinheit oder im Zylindermantel vorgesehen sein.

Nach dem Aufdosieren des Zylinders 2, bei welchem eine Substanz über die Zuleitung 4 durch die seitliche Öffnung 2a in den Zylinder 2 gelangt, wird der Zylinder 2 so gedreht, im Ausführungsbeispiel um 180 Grad um seine Längsachse, dass die einzige Öffnung 2a des Zylinders 2 in Verbindung mit der Ableitung 5 steht. In diesem Zustand kann der Kolben 3 wieder in den Zylinder 2 eingeschoben werden, um dadurch die in dem Zylinder 2 enthaltene Substanz zu verdrängen und durch die geöffnete selbst schließende Öffnung 2a des Zylinders 2 an die Ableitung 5 abzugeben, welche mit einem nicht gezeigten Infusionsset verbunden ist.

Anschließend kann der Zylinder 2 wieder zurückgedreht oder weitergedreht werden, bis die seitliche Öffnung 2a des Zylinders 2 wieder in Fluid-Verbindung mit der Zuleitung 4 steht, um erneut eine dosiert abzugebende Substanz durch die Zuleitung 4 aufzunehmen.

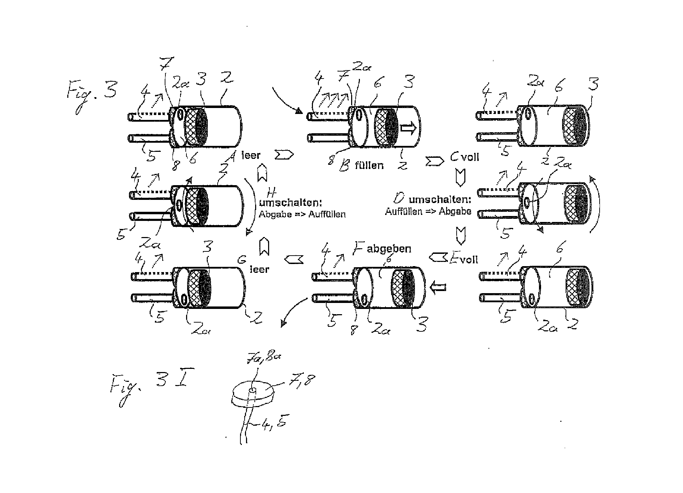

Figur 3 zeigt einen Kreisprozess, welcher einen Funktionsablauf beim Dosieren mit einer Ausführungsform der erfindungsgemäßen Dosiervorrichtung veranschaulicht. Ausgehend von einem mit A bezeichneten Anfangszustand, in welchem der Zylinder 2 leer ist und der Kolben 3 fast vollständig oder vollständig in den Zylinder 2 in Richtung auf die Öffnung 2a an der Zylinderstirnseite eingefahren ist, wird der Zylinder 2 durch die Zuleitung 4, welche luftdurchlässig ausgebildet ist, allmählich gefüllt, wie in Figur 3B gezeigt, wobei der Kolben 3 in der durch den Pfeil gezeigten Richtung innerhalb des Zylinders 2 so bewegt wird, dass sich das veränderbare Volumen 6 vergrößert, wodurch in dem Zylinder 2 ein Unterdruck erzeugt werden kann, wodurch die von einem Reservoir (nicht gezeigt) zuzuführende Substanz durch die Zuleitung 4 angesaugt oder in den Zylinder 2 eingebracht wird. Dabei ist der Zylinder 2 oder die Öffnung 2a so positioniert oder gedreht, dass diese möglichst vor der Öffnung der Zuleitung 4 liegt, so dass eine Fluidverbindung von der Zuleitung 4 zu dem veränderbaren Volumen 6 durch die Öffnung 2a geschaffen wird.

An der Vorderseite bzw. der Verbindungs- oder Kontaktseite der Zuleitung 4 und der Ableitung 5 mit dem Zylinder 2 sind in der gezeigten Ausführungsform Dichtelemente 7 und 8 vorgesehen, welche um die Öffnungen der Zuleitung 4 und der Ableitung 5 herum angeordnet sind und ein Austreten der in diesen Leitungen 4, 5 geführten Substanz oder Flüssigkeit verhindern sollen. Die Dichtungen 7 und 8 können z.B. aus scheibenförmigen oder ringförmigen Elementen bestehen, wie in Figur 3I gezeigt, welche zum Beispiel durch ein elastisches Material gebildet werden, wobei der Zylinder 2 oder eine Stirnseite des Zylinders 2 vorzugsweise leicht über die Dichtelemente 7 und 8 gleiten können.

Wie aus Figur 3C ersichtlich ist, befindet sich der Zylinder 2 im vollständig gefüllten Zustand, wenn der Kolben 3 bis zu einer Maximalposition herausgezogen wurde, wobei dieses Maximalvolumen der Dosiereinheit mit $VOL_{Zylinder, max}$ bezeichnet wird.

Anschließend wird der Zylinder 2 um seine Längsachse gedreht, wie in Figur 3D gezeigt, um von dem Auffüllvorgang des Zylinders 2 zu dem Abgabevorgang umzuschalten. Dabei wird die Drehung des Zylinders 2 so lange durchgeführt, bis die Öffnung 2a an der Abgabeleitung 5 liegt, wie in Figur 3E gezeigt, so dass eine Fluidverbindung zwischen dem veränderbaren Volumen 6 des Zylinders 2 und der Abgabeleitung 5 durch die Öffnung 2a hergestellt wird.

Wird nun der Kolben 3 wieder in den Zylinder 2 eingefahren, wie in Figur 3F gezeigt, so wird die in dem veränderbaren Volumen 6 enthaltene Substanz durch die Öffnung 2a und die Ableitung 5 z.B. an ein Infusionsset abgegeben. Dies kann solange durchgeführt werden, bis der Zylinder 2 vollständig oder fast vollständig entleert ist, wie in Figur 3G gezeigt. Dabei ist der Kolben 3 möglichst bis zu einem vorderen oder Abgabeende des Zylinders 2 in diesen eingefahren.

Anschließend kann, wie in Figur 3H gezeigt, die Dosiereinheit wieder umgeschaltet werden, um nach erfolgtem Abgabevorgang das veränderbare Volumen 6 wieder aufzufüllen. Zur Umschaltung der Dosiervorrichtung wird der Zylinder 2 wieder so gedreht, dass die Öffnung 2a wieder vor der Zuleitung 4 liegt, wie in Figur 3A gezeigt.

Das Hin- und Herdrehen oder Bewegen des Zylinders 2, wie in den Figuren 3D und 3H gezeigt, kann sowohl durch eine Drehung in die gleiche Richtung, also z.B. bezogen auf die Längsachse des Zylinders immer im Uhrzeigersinn oder immer gegen den Uhrzeigersinn, erfolgen. Alternativ ist es auch möglich, dass der Zylinder 2 zum Umschalten von einem Auffüllvorgang in den Abgabevorgang, wie in Figur 3D gezeigt, in eine erste Richtung, wie z.B. nach links, gedreht wird, und zum Umschalten von dem Abgabevorgang zum Auffüllvorgang, wie in Figur 3H gezeigt, in die entgegengesetzte Richtung, z.B. nach rechts, gedreht wird. Dies ist insbesondere dann vorteilhaft, wenn mit einem einzigen Motor eine Hin- und Herbewegung des Stopfens 3 realisiert werden soll, wobei der Motor auch noch zur Hin- und Herbewegung oder Drehung des Zylinders 2 verwendet wird.

Figur 3I zeigt in perspektivischer Ansicht ein Dichtelement 7, 8 mit der darin angeordneten Durchtrittsöffnung 7a bzw. 8a, an welche sich die Zuleitung 4 in den Zuständen gemäß Figuren 3A bis 3C bzw. die Ableitung 5 in den Zuständen gemäß Figuren 3E bis 3G anschließt.

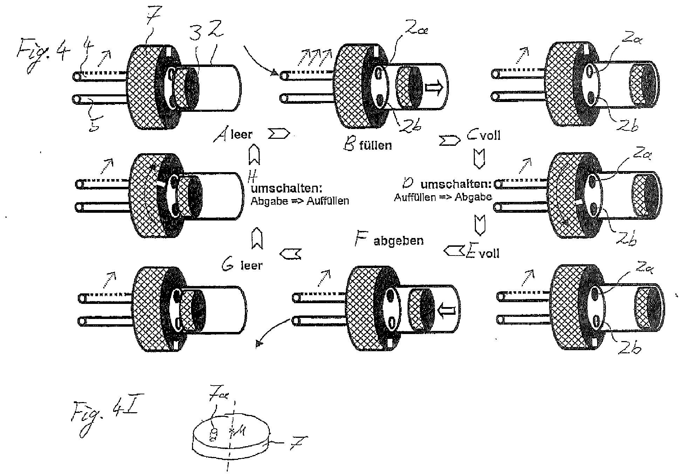

Figur 4 zeigt eine weitere Ausführungsform der Erfindung, wobei der Funktionsablauf ähnlich wie in Figur 3 ist und ein Zylinder 2 an seiner zum Befüllen und Abgeben dienenden Stirnseite Öffnungen 2a und 2b aufweist, welche z.B. bezogen auf einen Mittelpunkt der Stirnfläche des Zylinders 2 einander gegenüberliegend angeordnet sein können. Vor den Öffnungen 2a und 2b des Zylinders 2 ist ein Dichtelement 7 angeordnet, in welchem eine durchgehende Öffnung 7a vorgesehen ist; siehe Fig. 4I. Das Dichtelement 7 ist relativ zu dem Zylinder 2 und der Zuführleitung 4, sowie der Abführleitung 5, drehbar oder beweglich. Vorzugsweise befinden sich der Zylinder 2, die Zuführleitung 4 und die Abführleitung 5 in einem definierten oder festen Lageverhältnis. Durch die Drehung des Dichtelementes 7 mit der darin angeordneten Öffnung 7a können die Öffnungen 2a und 2b des Zylinders 2 entweder beide verschlossen werden, wie in den Umschaltvorgängen in den Figuren 4D und 4H gezeigt, oder alternierend geöffnet werden.

Während des Auffüllvorganges, wie in dem Ablauf der Figuren 4A, 4B und 4C gezeigt, befindet sich die Öffnung 7a des Dichtelementes 7 in einer Position, in welcher eine Fluidverbindung zwischen der Zuführleitung 4 und dem veränderbaren Volumen 6 hergestellt werden kann. Zum Umschalten zwischen dem Auffüllvorgang und dem Abgabevorgang wird das Dichtelement 7 um seine Mittelachse M gedreht, wie in Figur 4D gezeigt, bis die Öffnung 7a des Dichtelementes 7 so angeordnet ist, dass die Zuführöffnung 2a des Zylinders 2 verschlossen ist und die Abgabeöffnung 2b des Zylinders mit der Abgabeleitung 5 fluidisch gekoppelt ist, um den in den Figuren 4E, 4F und 4G gezeigten Abgabevorgang durchzuführen.

Anschließend kann von dem Abgabevorgang wieder in den Auffüllvorgang umgeschaltet werden, wie in Figur 4H gezeigt, wobei das Dichtelement 7 wieder so gedreht wird, dass die Öffnung 7a des Dichtelementes 7 in der in Figur 4A gezeigten Position ist.

Figur 4I zeigt in perspektivischer Ansicht das Dichtelement 7 mit einem von dem Mittelpunkt bzw. Drehpunkt M des Dichtelementes 7 in radiale Richtung versetzten Durchgang oder Durchleitungsstück 7a.

**Patentansprüche**

1. Dosiervorrichtung für ein Infusionssystem mit einer Dosiereinheit (2, 3), die ein Leck und ein unbeabsichtigtes oder unkontrolliertes Durchtreten einer Substanz verhindert, wobei die Dosiereinheit (2, 3) Folgendes aufweist:

   ein veränderbares Volumen (6),
   eine Zufuhröffnung (2a), die so öffen- und schließbar ist, dass die Zufuhr einer Substanz durch die Zufuhröffnung (2a) zum veränderbaren Volumen nur dann ermöglicht wird, wenn die Zufuhröffnung (2a) geöffnet ist,
   eine Abgabeöffnung (2b), die so öffen- und schließbar ist, dass die Abgabe der Substanz aus dem veränderbaren Volumen (2b) durch die Abgabeöffnung (2b) an eine Abgabeeinheit des Infusionssystems nur dann ermöglicht wird, wenn die Abgabeöffnung (2b) geöffnet ist,
   eine Zuführleitung (4);
   eine Abführleitung (5);
   **gekennzeichnet durch** ein vor der Zufuhröffnung (2a) und der Abgabeöffnung (2b) angeordnetes Dichtelement (7), in welchem eine durchgehende Öffnung (7a) vorgesehen ist;

   wobei das Dichtelement (7) drehbar ist, um einen ersten Zustand, einen zweiten Zustand oder einen dritten Zustand zu wählen, wobei das veränderbare Volumen im ersten Zustand mit der Zuführleitung (4), im zweiten Zustand weder mit der Zuführleitung (4) noch mit der Abführleitung (5) und im dritten Zustand mit der Abführleitung (5) in Fluidverbindung steht, und wobei die Zuführleitung nie über das veränderbare Volumen (6) mit der Abführleitung (5) in Fluidverbindung steht, so dass ein Leck und ein unbeabsichtigtes oder unkontrolliertes Durchtreten der Substanz aus der Zuführleitung (4) in die Abführleitung (5) verhindert wird.

2. Dosiervorrichtung nach Anspruch 1, wobei

   die Dosiereinheit (2, 3) als Zylinder (2) gestaltet ist und
   die Zufuhröffnung (2a) und die Abgabeöffnung (2b) am Zylinder (2) angeordnet sind.

3. Dosiervorrichtung nach Anspruch 2, wobei die Zufuhröffnung (2a) und die Abgabeöffnung (2b) an einer Seite des Zylinders (2) angeordnet sind.

4. Dosiervorrichtung nach Anspruch 2, wobei der Zylinder (2) einen in dem Zylinder (2) beweglichen Kolben (3) zur Volumenvergrößerung oder -verkleinerung des veränderbaren Volumens (6) aufweist.

5. Dosiervorrichtung nach Anspruch 1, die ferner mindestens ein Ventil aufweist, wobei das mindestens eine Ventil in der Dosiereinheit (2, 3), in der Zufuhröffnung (2a), in der Abgabeöffnung (2b), an der Zuführleitung (4) oder an der Abführleitung (5) angeordnet ist.

6. Dosiervorrichtung nach Anspruch 1, die ferner mindestens einen zum Drehen des Dichtelements (7) ausgelegten Motor aufweist, um das Dichtelement (7) zu drehen.

7. Dosiervorrichtung nach Anspruch 1, wobei die Dosiereinheit (2, 3), die Zuführleitung (4), die Abführleitung (5) oder eine beliebige Kombination davon ein luftdurchlässiges Material aufweist.

8. Dosiervorrichtung nach Anspruch 1, wobei im Dichtelement (7) eine Dichtelementöffnung (7a) vorgesehen ist, wobei die Dichtelementöffnung (7a) an einer Position angeordnet ist, an der die Fluidverbindung zwischen dem veränderbaren Volumen (6) und der Zuführleitung (4) im ersten Zustand herstellbar ist, und wobei die Dichtelementöffnung (7a) in einer Position angeordnet ist, an der die Fluidverbindung zwischen dem veränderbaren Volumen (6) und der Abführleitung (5) im dritten Zustand herstellbar ist.

9. Dosiervorrichtung nach Anspruch 8, wobei die Zuführöffnung (2a) und die Abführöffnung (2b) beide verschlossen oder abwechselnd geöffnet werden können.

10. Dosiervorrichtung nach Anspruch 1, die ferner einen Vorratsbehälter aufweist, der mit der Zuführleitung (4) in Fluidverbindung steht.

11. Dosiervorrichtung nach einem der vorhergehenden Ansprüche, die ferner einen Sensor oder Detektor zum Detektieren eines Lecks, einer Okklusion, eines Drucks, einer Kraft oder von Blasen bei der Dosiervorrichtung aufweist.

## Claims

1. A dosing device for an infusion system having a dosing unit (2, 3) preventing leakage and unintentional or uncontrolled passage of a substance, wherein the dosing unit (2, 3) has the following:

   a variable volume (6),
   a supply port (2a) openable and closable so as to allow supply of a substance through the supply port (2a) to the variable volume only when the supply port (2a) is opened,
   a discharge port (2b) openable and closable so as to allow discharge of the substance from the variable volume (2b) through the discharge port (2b) to a discharge unit of the infusion system only when the discharge port (2b) is opened,
   a supply line (4);
   a discharge line (5);
   **characterised by** a sealing element (7) arranged in front of the supply port (2a) and the discharge port (2b), in which a through-opening (7a) is provided;

   wherein the sealing element (7) is rotatable to select a first state, a second state or a third state, wherein the variable volume is in fluid communication with the supply line (4) in the first state, neither with the supply line (4) nor with the discharge line (5) in the second state, and with the discharge line (5) in the third state, and wherein the supply line is never in fluid communication with the discharge line (5) via the variable volume (6) so as to prevent leakage and unintentional or uncontrolled passage of the substance from the supply line (4) into the discharge line (5).

2. The dosing device according to claim 1, wherein

   the dosing unit (2, 3) is configured as a cylinder (2), and
   the supply port (2a) and the discharge port (2b) are arranged on the cylinder (2).

3. The dosing device according to claim 2, wherein the supply port (2a) and the discharge port (2b) are arranged on one side of the cylinder (2).

4. The dosing device according to claim 2, wherein the cylinder (2) comprises a piston (3) movable in the cylinder (2) for increasing or decreasing the volume of the variable volume (6).

5. The dosing device according to claim 1, further having at least one valve, wherein the at least one valve is arranged in the dosing unit (2, 3), in the supply port (2a), in the discharge port (2b), on the supply line (4) or on the discharge line (5).

6. The dosing device according to claim 1, further having at least one motor configured to rotate the sealing element (7) in order to rotate the sealing element (7).

7. The dosing device according to claim 1, wherein the dosing unit (2, 3), the supply line (4), the discharge line (5) or any combination thereof comprises an air-permeable material.

8. The dosing device according to claim 1, wherein a sealing element opening (7a) is provided in the sealing element (7), wherein the sealing element opening (7a) is arranged at a position where fluid communication between the variable volume (6) and the supply line (4) can be established in the first state, and wherein the sealing element opening (7a) is arranged at a position where fluid communication between the variable volume (6) and the discharge line (5) can be established in the third state.

9. The dosing device according to claim 8, wherein the supply port (2a) and the discharge port (2b) can both be closed or opened in an alternating manner.

10. The dosing device according to claim 1, further having a reservoir in fluid communication with the supply line (4).

11. The dosing device according to any one of the preceding claims, further having a sensor or detector for detecting a leak, an occlusion, a pressure, a force or bubbles in the dosing device.

**Revendications**

1. Dispositif de dosage pour un système de perfusion, comprenant une unité de dosage (2, 3), laquelle empêche une fuite et un passage involontaire ou incontrôlé d'une substance, dans lequel l'unité de dosage (2, 3) comporte :

   un volume (6) variable,
   une ouverture d'alimentation (2a), laquelle peut être ouverte et fermée de telle sorte que l'alimentation d'une substance par l'ouverture d'alimentation (2a) vers le volume variable n'est possible que lorsque l'ouverture d'alimentation (2a) est ouverte,
   une ouverture d'évacuation (2b), laquelle peut être ouverte et fermée de telle sorte que l'évacuation de la substance du volume (2b) variable par l'ouverture d'évacuation (2b) vers une unité d'évacuation d'un système de perfusion n'est possible que lorsque ladite ouverture d'évacuation (2b) est ouverte,
   une conduite d'alimentation (4) ;
   une conduite d'évacuation (5) ;
   **caractérisé par** un élément d'étanchéité (7) disposé devant l'ouverture d'alimentation (2a) et l'ouverture d'évacuation (2b), dans lequel est fournie une ouverture (7a) traversante ;

   dans lequel l'élément d'étanchéité (7) est rotatif pour sélectionner un premier état, un deuxième état ou un troisième état, dans lequel le volume variable est en communication fluidique, dans le premier état, avec la conduite d'alimentation (4), dans le deuxième état, ni avec la conduite d'alimentation (4) ni avec la conduite d'évacuation (5), dans le troisième état, avec la conduite d'évacuation (5), et dans lequel la conduite d'alimentation n'est jamais en communication fluidique avec la conduite d'évacuation (5) par l'intermédiaire du volume (6) variable de telle sorte qu'une fuite et un passage involontaire ou incontrôlé de la substance de la conduite d'alimentation (4) vers la conduite d'évacuation (5) sont empêchés.

2. Dispositif de dosage selon la revendication 1, dans lequel

   l'unité de dosage (2, 3) est conçue sous la forme d'un cylindre (2) et
   l'ouverture d'alimentation (2a) et l'ouverture d'évacuation (2b) sont disposées sur le cylindre (2).

3. Dispositif de dosage selon la revendication 2, dans lequel l'ouverture d'alimentation (2a) et l'ouverture d'évacuation (2b) sont disposées sur un côté du cylindre (2).

4. Dispositif de dosage selon la revendication 2, dans lequel le cylindre (2) comporte un piston (3) mobile dans le cylindre (2) pour augmenter ou diminuer le volume du volume (6) variable.

5. Dispositif de dosage selon la revendication 1, comprenant en outre au moins une vanne, dans lequel l'au moins une vanne est disposée dans l'unité de dosage (2, 3), dans l'ouverture d'alimentation (2a), dans l'ouverture d'évacuation (2b), sur la conduite d'alimentation (4) ou sur la conduite d'évacuation (5).

6. Dispositif de dosage selon la revendication 1, comprenant en outre au moins un moteur conçu pour faire tourner l'élément d'étanchéité (7), pour faire tourner l'élément d'étanchéité (7).

7. Dispositif de dosage selon la revendication 1, dans lequel l'unité de dosage (2, 3), la conduite d'alimentation (4), la conduite d'évacuation (5) ou toute combinaison de celles-ci, comportent une matière perméable à l'air.

8. Dispositif de dosage selon la revendication 1, dans lequel une ouverture d'élément d'étanchéité (7a) est fournie dans l'élément d'étanchéité (7), dans lequel l'ouverture d'élément d'étanchéité (7a) est disposée à un emplacement auquel la communication fluidique entre le volume (6) variable et la conduite d'alimentation (4) peut être établie dans le premier état, et dans lequel l'ouverture d'élément d'étanchéité (7a) est disposée à un emplacement auquel la communication fluidique entre le volume (6) variable et la conduite d'évacuation (5) peut être établie dans le troisième état.

9. Dispositif de dosage selon la revendication 8, dans lequel l'ouverture d'alimentation (2a) et l'ouverture d'évacuation (2b) peuvent être toutes les deux fermées ou ouvertes en alternance.

10. Dispositif de dosage selon la revendication 1, comprenant en outre un réservoir, lequel est en communication fluidique avec la conduite d'alimentation (4).

11. Dispositif de dosage selon l'une quelconque des revendications précédentes, lequel comprend en outre un capteur ou un détecteur pour détecter une fuite, une occlusion, une pression, une force ou des bulles dans le dispositif de dosage.

Fig. 1A    Fig. 1B    Fig. 1C

fill / degas    rotate    expel

Fig. 2

Fig. 3

leer
füllen
voll

umschalten:
Abgabe => Auffüllen

umschalten:
Auffüllen => Abgabe

leer
abgeben
voll

Fig. 3 I

EP 3 258 223 B1

Fig. 4

A leer ⟹    B füllen    ⟹ C voll

H umschalten:
Abgabe => Auffüllen

D umschalten:
Auffüllen => Abgabe

F abgeben    ⟸ E voll

G leer ⟸

Fig. 4I

**IN DER BESCHREIBUNG AUFGEFÜHRTE DOKUMENTE**

**In der Beschreibung aufgeführte Patentdokumente**

- CH 688224 A5
- US 6010485 A
- US 6749587 B2
- US 4643723 A
- WO 9304714 A
- EP 0600948 B1
- US 20040069044 A1
- US 5207666 A
- US 20050159708 A1
- EP 1633417 B1
- US 5807321 A
- WO 2007000064 A1
- EP 0980690 A2
- US 5961303 A